# EUROPEAN PATENT APPLICATION

(11) **EP 4 516 905 A1**
(43) Date of publication of application: **05.03.2025**
(21) Application number: 23796450.7
(22) Date of filing: 26.04.2023
(51) Int. Cl.: C12N 15/113, C12N 15/09, C12N 5/10, A61K 45/00, A61K 48/00, A61K 31/713, A61P 9/00

(54) **EPICARDIAL CELL REGENERATION PROMOTER AND METHOD FOR PROMOTING EPICARDIAL CELL REGENERATION**

(30) Priority: 27.04.2022 JP 2022073500
(71) Applicant: Kyoto University, Kyoto-shi, Kyoto 606-8501 (JP)
(72) Inventor: YOSHIDA, Yoshinori, Kyoto-shi, Kyoto 606-8501 (JP); LUCENA-CACACE, Antonio, Kyoto-shi, Kyoto 606-8501 (JP); TIAN, Yu, Kyoto-shi, Kyoto 606-8501 (JP)
(74) Representative: Hoffmann Eitle
(86) International application number: PCT/JP2023/016537
(87) International publication number: WO 2023/210713

(57) **Abstract**

The present invention provides a drug which can be used as a pharmaceutical product for promoting epicardial cell regeneration and treating cardiac injury. In the present invention, a p21-inhibiting substance is used as an agent for promoting epicardial cell regeneration.

## Description

### TECHNICAL FIELD

The present invention relates to an agent for promoting epicardial cell regeneration and a pharmaceutical product for treating cardiac injury containing the same. Further, the present invention relates to a method for promoting epicardial cell regeneration, and a method for producing epicardial cells having an enhanced regenerative capacity.

### BACKGROUND ART

The regenerative capacity of the heart after injury varies depending on the species. Neonatal mice are capable of regenerating their hearts, and their cardiac function recovers even after injury. However, this regenerative capacity is lost after birth. Such a regenerative capacity is totally absent in humans. The epicardium covering the heart has a function indispensable for cardiac regeneration. In species capable of cardiac regeneration during the neonatal period, the epicardium transitions from a non-proliferative state to a reactivated state upon cardiac injury. However, the epicardium of an adult human hardly has a proliferative capacity, permanently.

So far, there are a number of approaches for differentiating epicardial cells derived from human pluripotent stem (iPS) cells (see, for example, Non-patent Document 1). However, these approaches are neither intended to promote the regeneration of the epicardium, nor to explore the possibility of regenerating epicardial cells derived from human iPS cells for clinical purposes. In other words, most of the approaches focus on the differentiation, and the capacity of the epicardial cells derived from human iPS cells are maintained for the evaluation of cell properties in these approaches; however, promoting epicardial cell regeneration has never been reported.

So far, it has been suggested that the cyclin-dependent kinase inhibitor p21 has an impact on epimorphic regeneration and the like, and is involved in liver regeneration. However, there is no report on the effect of p21 on the regenerative capacity of epicardial cells. Since p21 is involved in a number of cell processes in a complex manner, it is hard to predict how a decrease in the protein level of p21 affects a specific tissue or cells.

### RELATED ART DOCUMENT

### NON-PATENT DOCUMENT

Nonpatent Document 1: Junghof J, et al. NPJ Regen Med. 2022 Feb 2; 7 (1): 14. doi: 10. 1038/s41536-022-00207-w.

### SUMMARY OF THE INVENTION

### PROBLEMS TO BE SOLVED BY THE INVENTION

An object of the present invention is to provide a drug for promoting epicardial cell regeneration. More particularly, an object of the present invention is to provide a drug for promoting epicardial cell regeneration and treating cardiac injury. Another object of the present invention is to provide a method for promoting epicardial cell regeneration.

### MEANS FOR SOLVING THE PROBLEMS

As a result of intensive studies to solve the above-mentioned problems, the present inventors have found out that p21 is an important factor that inhibits the reactivation of epicardial cells, and that the use of a p21-inhibiting substance makes it possible to promote epicardial cell regeneration and to provide a therapeutic effect such as repairing the injured cardiac tissue, thereby completed the present invention.

[1] An agent for promoting epicardial cell regeneration containing a p21-inhibiting substance.
[2] The agent for promoting epicardial cell regeneration according to [1], wherein the p21-inhibiting substance is a substance that inhibits the expression of CDKN1A gene.
[3] The agent for promoting epicardial cell regeneration according to [2], wherein the substance that inhibits the expression of the CDKN1A gene is a nucleic acid containing a siRNA sequence that targets the CDKN1A gene.
[4] The agent for promoting epicardial cell regeneration according to [3], wherein the siRNA sequence includes a sense strand having the sequence of SEQ ID NO: 1, and an anti-sense strand having the sequence of SEQ ID NO: 2.
[5] The agent for promoting epicardial cell regeneration according to [1] or [2], wherein the p21-inhibiting substance contains a guide RNA for a CRISPR-Cas system, and the guide RNA for the CRISPR-Cas system is a guide RNA designed to target the CDKN1A gene.
[6] A pharmaceutical composition for treating cardiac injury, the composition including the agent for promoting epicardial cell regeneration according to any one of [1] to [5].
[7] A method for promoting epicardial cell regeneration in vitro, the method including the step of inhibiting p21 in the epicardial cells.
[8] The method according to [7], wherein the step of inhibiting p21 is carried out by inhibiting the expression of CDKN1A gene.
[9] The method according to [8], wherein the inhibition of the expression of the CDKN1A gene is achieved by introducing a siRNA into the epicardial cells.
[10] The method according to [8], wherein the step of inhibiting p21 is carried out by introducing a guide RNA designed to target the CDKN1A gene and a CRISPR enzyme into the epicardial cells.
[11] A method for producing epicardial cells having an enhanced regenerative capacity, the method comprising:
   A) the step of providing epicardial cells; and
   B) the step of inhibiting p21 in the epicardial cells provided in the step A).
[12] The method according to [11], wherein the epicardial cells provided in the step A) are epicardial cells obtained by inducing differentiation from pluripotent stem cells.
[13] A p21-inhibiting substance for promoting epicardial cell regeneration.
[14] A use of a p21-inhibiting substance in the production of an agent for promoting epicardial cell regeneration.
[15] A p21-inhibiting substance for treating cardiac injury.
[16] A use of a p21-inhibiting substance in the production of a pharmaceutical product for treating cardiac injury.
[17] A method for promoting epicardial cell regeneration in a subject in need thereof, the method including the step of administering an effective amount of a p21-inhibiting substance to the subject.
[18] A method for treating cardiac injury in a subject in need thereof, the method including the step of administering an effective amount of a p21-inhibiting substance to the subject.

### EFFECT OF THE INVENTION

The present invention enables the promotion of epicardial cell regeneration. As a result, it is possible to promote epicardial cell regeneration in injured cardiac tissue, and further, to induce myocardial repair and regeneration, and to enhance the regenerative capacity of the injured heart, thereby enabling the treatment of cardiac injury.

### BRIEF DESCRIPTION OF THE DRAWINGS

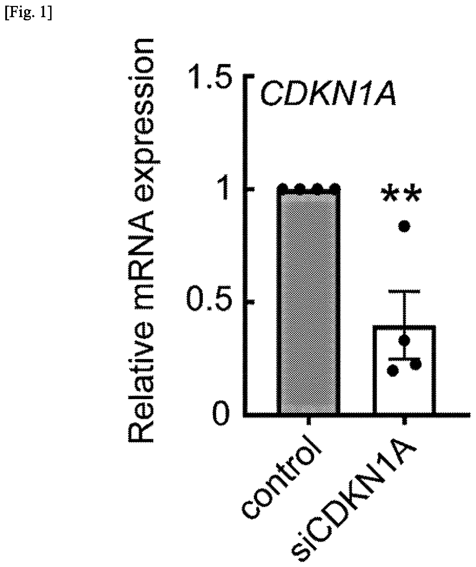

FIG. 1 is a graph showing the relative mRNA expression levels of CDKN1A, after the siRNA transfection assay in human epicardial cells obtained by inducing differentiation from a human iPS cell line. The analysis was performed in biological triplicates. The symbol "**" indicates P < 0.01 as compared to control conditions.

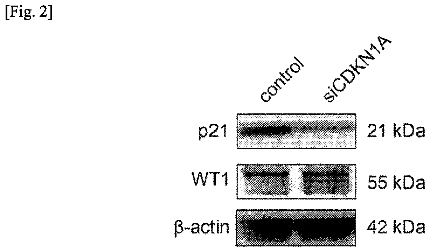

FIG. 2 shows photographs illustrating the results of the Western blotting analysis of p21 protein and WT1 protein, after the siRNA transfection assay in the human epicardial cells.

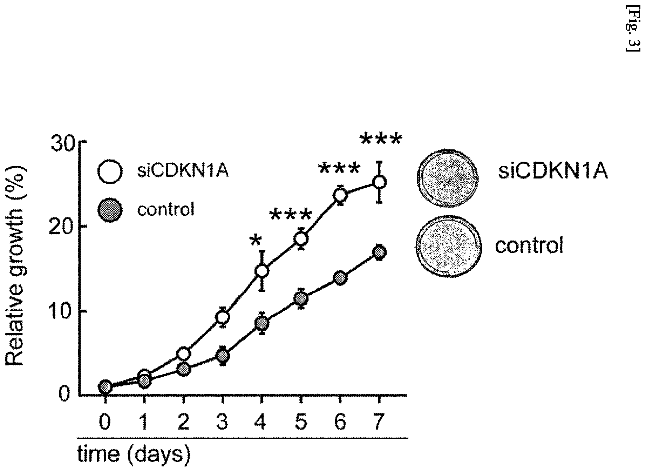

FIG. 3 is a graph showing cell proliferation curves for 7 days after the siRNA transfection assay in the human epicardial cells. The photographs on the right show the cells on the dishes imaged on day 7. The analysis was performed in biological triplicates. The symbol "*" indicates P < 0.05, and the symbol "***" indicates P < 0.001 as compared to the control conditions.

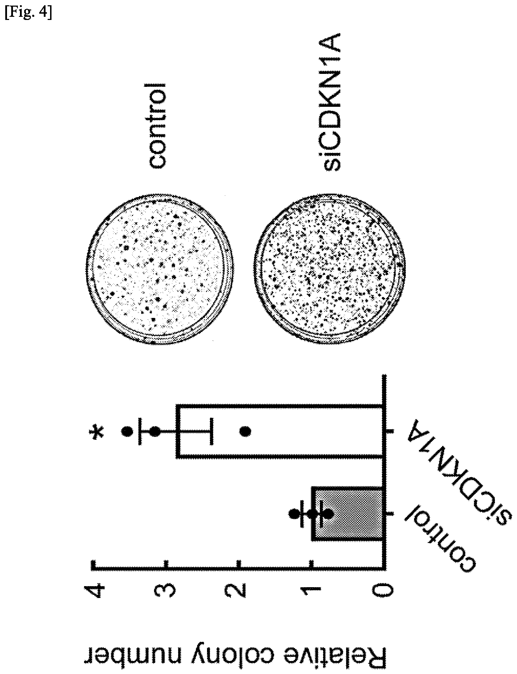

FIG. 4 is a graph showing the results of the colony formation assay in the human epicardial cells, in which colony formation was carried out for 14 days after the siRNA transfection assay. The photographs on the right show the colonies on the dishes imaged on day 14. The analysis was performed in biological triplicates. The symbol "*" indicates P < 0.05 as compared to the control conditions.

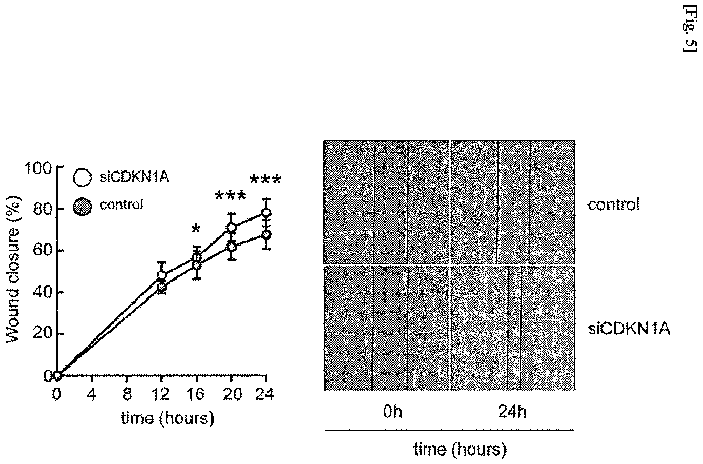

FIG. 5 shows a graph illustrating the wound closure rate during the period of time from 0 to 24 hours after scratching the human epicardial cells, in the wound-healing assay, and photographs illustrating the state of wound closure 24 hours after scratching. The analysis was performed in biological triplicates. The symbol "*" indicates P < 0.05, and the symbol "***" indicates P < 0.001 as compared to the control conditions.

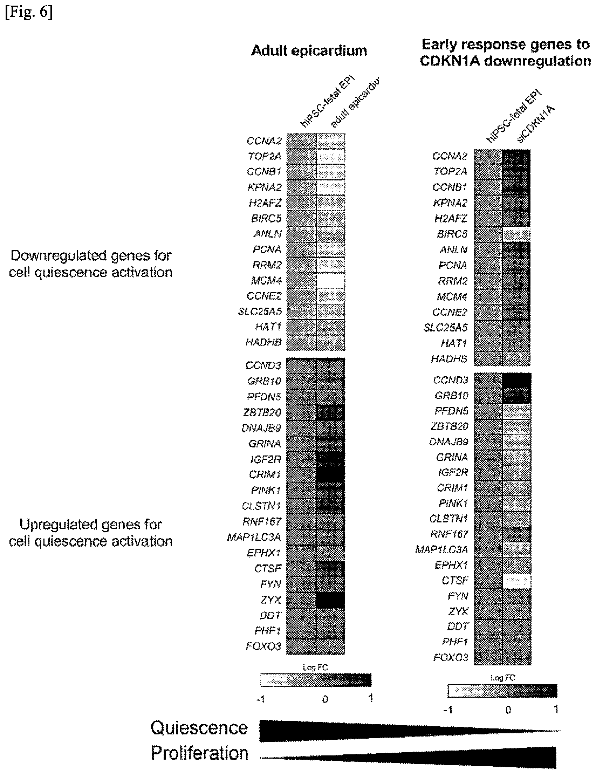

FIG. 6 is a diagram showing the results of the transcriptome analysis for detecting the gene signature of cell quiescence, by RNA sequencing. The comparison of the human iPS cell-derived fetal epicardium and the adult epicardium (GSE84085), as well as the comparison of the human iPS cell-derived fetal epicardium and the siCDKN1A group, are represented by heatmaps showing the relative mRNA expression levels (the log ratio of fold change: log FC), to detect cell quiescence.

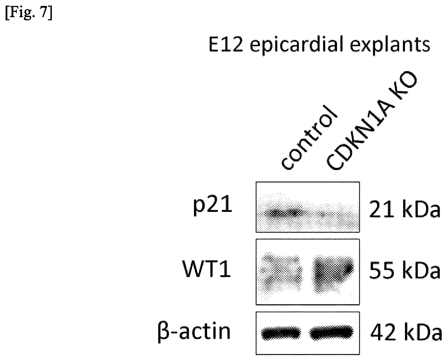

FIG. 7 is a diagram showing the results of the Western blotting analysis of the p21 protein and the WT1 protein, in epicardial explants derived from the 12-day-old embryos (E12) of a CDKN1A knockout mouse and a control mouse (C57BL/6J mouse).

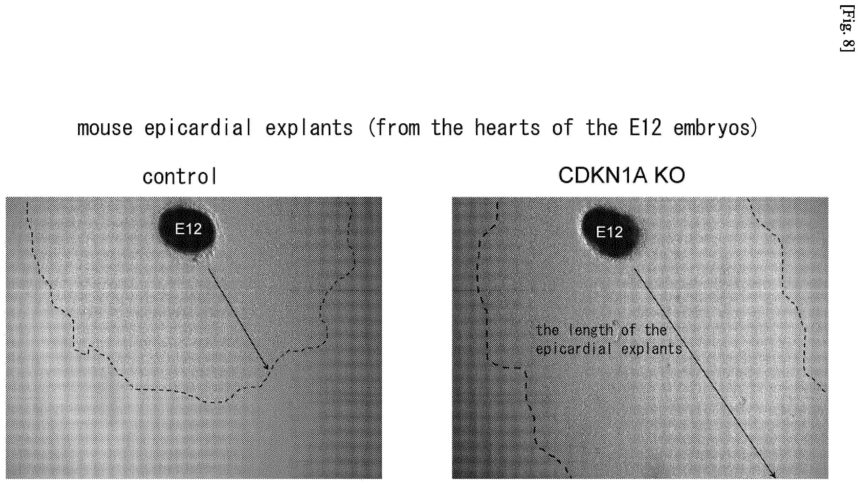

FIG. 8 shows photographs illustrating the results of the ex vivo explant assay of the epicardial explants derived from the hearts of the E12 embryos of the CDKN1A knockout mouse and the control mouse, in which the proliferation of the epicardial cells and the length of the explants were observed by a microscope 7 days after the culture.

### MODE FOR CARRYING OUT THE INVENTION

The present invention will be described below in detail.

### <1> Agent for Promoting Epicardial Cell Regeneration

The present invention relates to an agent for promoting epicardial cell regeneration containing a p21-inhibiting substance.

The addition of a p21-inhibiting substance to epicardial cells enables the regenerative capacity which is stopped in adult epicardial cells to be reactivated, making it possible to promote epicardial cell regeneration.

Epicardial cells are cells covering the surface of the myocardium, and are characterized by the expression of markers such as WT1 (Wilms Tumor 1), TBX18 (T-Box Transcription Factor 18) and ALDH1A2 (Aldehyde Dehydrogenase 1 Family Member A2).

The term "epicardial cell regeneration promotion" is characterized, for example, by an improved viability of epicardial cells, an improved proliferative capacity of epicardial cells, an improved tissue repair capacity of the epicardium and/or an improved expression of a gene involved in the reactivation of the regenerative capacity of the epicardium.

The viability of epicardial cells can be indicated, for example, by culturing epicardial cells, and measuring the viability (the percentage of live cells) after the passage of a certain period of time. It can be determined that the viability of the epicardial cells is improved, in cases where the viability of the epicardial cells is increased when a p21-inhibiting substance is added to the culture medium, as compared to the case in which the p21-inhibiting substance is not added to the culture medium. The period of time for culturing the epicardial cells at this time, may be, for example, 14 days.

The proliferative capacity of epicardial cells is indicated, for example, by culturing epicardial cells, and counting the number of cells over time in a certain period of time to generate a proliferation curve. It can be determined that the proliferative capacity of the epicardial cells is improved, in cases where the inclination of the proliferation curve is increased when a p21-inhibiting substance is added to the culture medium, as compared to the case in which the p21-inhibiting substance is not added to the culture medium. The period of time for culturing the epicardial cells at this time, may be, for example, 7 days. The number of cells over time may be counted, specifically, for example, every day.

The tissue repair capacity of the epicardium can be indicated, for example, by a wound-healing assay. Specifically, in the wound-healing assay, for example, epicardial cells are cultured until confluent, then some of the cells are physically scraped to cause a simulated wound, and the tissue repair capacity is indicated by the degree of the wound repaired after the passage of a certain period of time. It can be determined that the tissue repair capacity of the epicardium is improved, in cases where the degree of repair of the epicardial cells is improved when a p21-inhibiting substance is added to the culture medium, as compared to the case in which the p21-inhibiting substance is not added to the culture medium.

Examples of the gene involved in the reactivation of the regenerative capacity of the epicardium include: WT1 and TBX18 which are transcription factors; and ALDH1A2 which is an aldehyde dehydrogenase. It can be determined that the expression of a gene involved in the reactivation of the regenerative capacity of the epicardium is improved, in cases where the expression level of such a gene is increased when the epicardial cells are cultured with a p21-inhibiting substance added to the culture medium, as compared to the case in which the cells are cultured without adding the p21-inhibiting substance to the culture medium. The gene expression level can be evaluated, for example, by a known method such as RT-PCR.

p21 is known as a cyclin-dependent kinase inhibitor. p21 is a protein also referred to as CDKN1A, and is expressed from the CDKN1A gene locus. In the present invention, the p21 protein is also simply referred to as p21, and the gene that expresses p21 is also referred to as the CDKN1A gene or CDKN1A. p21 is known to have a function of binding to a complex of cyclin and the cyclin-dependent kinase 2 or 4 to inhibit its activity, and controlling the cell cycle progression in the G1 phase of the cell cycle.

The p21 protein and the CDKN1A gene are not particularly limited, and can be selected based on the epicardial cells whose regeneration is to be promoted. In the case of promoting the regeneration of human epicardial cells, it is preferred to use a substance that inhibits the human p21 protein or the human CDKN1A gene.

One example of the amino acid sequence of the human p21 protein is shown in SEQ ID NO: 10, and one example of the nucleotide sequence of the human CDKN1A gene is shown in SEQ ID NO: 9.

In the present invention, the definition of the phrase "inhibition of p21" includes the inhibition of the expression and the inhibition of the activity, of p21.

The inhibition of the expression of p21 refers to decreasing the transcription level (mRNA level) or the translation level (protein level) of the gene (CDKN1A) that encodes the p21 protein.

The inhibition of the expression of p21 can be confirmed by comparing the expression levels of the p21 protein or the CDKN1A gene per certain number of epicardial cells before and after the addition of a p21 inhibitor. The expression level of the p21 protein or the CDKN1A gene needs to be decreased as compared to the expression level before the addition of the p21 inhibitor. However, for example, the expression level is preferably decreased to 50% or less, 20% or less or 10% or less, of the expression level before the addition of the p21 inhibitor, and the expression level may be disappeared, decreasing to less than the detection limit level.

The expression of the p21 protein or the gene (CDKN1A) that encodes the p21 protein can be measured by Western blotting, RT-PCR, or the like.

The inhibition of the expression of p21 can be achieved, for example, by the operation of decreasing the expression of the gene (CDKN1A gene) that encodes the p21 protein.

The inhibition of the expression of the CDKN1A gene is achieved, for example, by introducing a mutation that causes a decrease in the transcription efficiency or the translation efficiency into the CDKN1A gene, manipulating a small molecule involved in the control of the transcription or translation, manipulating a nucleic acid such as a siRNA that triggers RNA interference, or the like, but not particularly limited thereto.

Further, the inhibition of the expression of the CDKN1A gene can be achieved by disrupting the CDKN1A gene. The disruption of a gene refers to modifying the gene so that a normally functioning protein is not produced from the gene. The definition of the fact that a normally functioning protein is not produced from the gene, includes the case in which no protein is produced from the gene at all, and the case in which a protein whose function per molecule is decreased or disappeared is produced from the gene.

The disruption of a gene or the introduction of a mutation may be carried out, for example, by using a CRISPR-Cas system. Specifically, the disruption of a gene or the introduction of a mutation may be carried out, for example, by using a guide RNA designed for the modification of a gene or the introduction of a mutation, and a CRISPR enzyme. Further, a DNA fragment for homologous recombination may be used, at the same time.

The p21-inhibiting substance is a substance for achieving the above described "inhibition of p21". Specifically, the p21-inhibiting substance may be, for example, a substance that inhibits the activity of p21, a nucleic acid such as a siRNA that targets the CDKN1A gene and triggers RNA interference, a guide RNA for a CRISPR-Cas system targeting the CDKN1A gene, or a CRISPR enzyme.

Specific examples of the nucleic acid such as a siRNA that triggers RNA interference include: siRNAs, shRNAs and miRNAs; and precursors thereof. In other words, the p21-inhibiting substance may be: a nucleic acid such as a siRNA, a shRNA or a miRNA that targets the CDKN1A gene, or a precursor thereof; or a DNA containing a sequence for expressing such an RNA.

The sequence of the nucleic acid such as a siRNA that induces the inhibition of the expression of the CDKN1A gene, can be designed by a known method, based on the sequence of the CDKN1A gene.

Further, the sequence of the guide RNA that induces the inhibition of the expression of the CDKN1A gene by the CRISPR-Cas system, can be designed by a known method, based on the sequence of the CDKN1A gene.

Specifically, the p21-inhibiting substance may be, for example: a siRNA containing a sense strand containing the nucleotide sequence of SEQ ID NO: 1 and an anti-sense strand containing the nucleotide sequence of SEQ ID NO: 2, or an shRNA containing a sequence contained in any of these sequences, as a core sequence; or a DNA containing a sequence for expressing any of these RNAs.

Examples of the method for introducing a nucleic acid into a cell or tissue include a method using a virus vector, and lipofection. Therefore, in cases where the p21-inhibiting substance is a nucleic acid such as an RNA or a DNA, the p21-inhibiting substance may be present in the form of a complex for use in a virus vector or for lipofection. Any known vector can be used as the virus vector, and examples thereof include adenovirus vectors, adeno-associated virus vectors and Sendai virus vectors.

The substance that inhibits the activity of the p21 protein may be, for example, an antibody (including a partial fragment thereof) against the p21 protein, or a compound that binds to the p21 protein to inhibit the activity of the p21 protein.

The inhibition of the activity of the p21 protein can be confirmed by comparing the activity of the p21 protein per certain number of epicardial cells before and after the addition of a p21 inhibitor. The activity of the p21 protein needs to be decreased as compared to the cells before the addition of the p21 inhibitor. However, for example, the activity of the p21 protein is preferably decreased to 50% or less, 20% or less, or 10% or less, as compared to the cells before the addition of the p21 inhibitor, and the activity may be completely disappeared.

The antibody against the p21 protein can be obtained by a known method, using the p21 protein or a partial peptide thereof (for example, a partial peptide of a region on the C terminal side of p21), as an antigen. Alternatively, a commercially available anti-p21 antibody may be used.

The p21-inhibiting substance can be added at a concentration capable of inhibiting the expression or the function of p21.

The p21-inhibiting substance is not particularly limited, as long as the substance inhibits the expression or the function of p21. Specific examples of the p21-inhibiting substance include: p21 antibodies, p21 siRNAs, p21 shRNAs, p21-antisense compounds; and 2-(2-chlorophenyl)-5,7-dihydroxy-8-[(3S, 4R)-3-hydroxy-1-methylpiperidin-4-yl]-4H-chromen-4-one (flavopiridol), (1R,2R,4S)-4-[(2R)-2-[(1R,9S,12S,15R,16E,18R,19R,21R,23S,24E,26E,28E,30S,32S,35R)-1,18-dihydroxy-19,30-dimethoxy-15,17,21,23,29,35-hexamethyl-2,3,10,14,20-pentaoxo-11,36-dioxa-4-azatricyclo [30.3.1.0^{4,9}] hexatriaconta-16,24,26,28-tetraen-12-yl]propyl]-2-methoxycyclohexyl-3-hydroxy-2-(hydroxymethyl)-2-methylpropanoate (temsirolimus), (3R,4S,5S,6R,7R,9R,11S,12R,13S,14R)-6-{[(2S,3R,4S,6R)-4-(dimethylamino)-3-hydroxy-6-methyloxan-2-yl]oxy}-14-ethyl-7,12,13-trihydroxy-4-{[(2R,4R,5S,6S)-5-hydroxy-4-methoxy-4,6-dimethyloxan-2-yl]oxyl-3,5,7,9,11,13-hexamethyl-10-(2,4,7-trioxa-1-azaoctan-1-ylidene)-1-oxacyclotetradecane-2-one (roxithromycin), 6-hydroxy-2-(4-hydroxyphenyl)benzo[b]thien-3-yl][4-[2-(1-piperidinyl)ethoxy]phenyl]methanone hydrochloride (raloxifene hydrochloride), (7S,9E,11S,12R,13S,14R,15R,16R,17S,18S,19E,21Z)-2,15,17,27,29-pentahydroxy-11-methoxy-3,7,12,14,16,18,22-heptamethyl-26-{(E)-[(4-methylpiperazin-1-yl)imino]methyl}-6,23-dioxo-8,30-dioxa-24-azatetracyclo[23.3.1.14'7.05'28]triaconta-1(28),2,4,9,19,21,25(29),26-octaen-13-yl acetate (rifampin), [(8R,9S,10R,13S,14S,17R)-17-acetyl-6,10,13-trimethyl-3-oxo-2,8,9,11,12,14,15,16-octahydro-1H-cyclopenta[a]phenanthren-17-yl]acetate

(megestrol acetate), 8-(4-amino-1-methylbutylamino)-6-methoxyquinoline diphosphate (primaquine diphosphate), potassium;[2-butyl-5-chloro-3-[[4-[2-(1,2,3-triaza-4-azanidacyclopenta-2,5-dien-5-yl)phenyl]phenyl]methyl]imidazol-4-yl]methanol (losartan potassium), (2S)-3-methyl-2-[pentanoyl-[[4-[2-(2H-tetrazol-5-yl)phenyl]phenyl]methyl]amino]butanoic acid (valsartan), (Z)-buto-2-ene-dioic acid;2-(2,2-dicyclohexylethyl)piperidine (perhexiline maleate), and 3-O-methyl-5-O-(2-methylpropyl)-2,6-dimethyl-4-(2-nitrophenyl)-1,4-dihydropyridine-3,5-dicarboxylate (nisoldipine).

### < Pharmaceutical Product for Treating Cardiac Injury >

Epicardial cells have a function indispensable for cardiac regeneration after injury. Therefore, the agent for promoting epicardial cell regeneration according to the present invention can be used as a pharmaceutical product for treating cardiac injury, by reactivating the regenerative capacity of the epicardium, and acquiring the regenerative capacity of the heart after injury. Accordingly, the present invention provides a pharmaceutical product for treating cardiac injury, which contains a p21-inhibiting substance as an active ingredient.

The p21-inhibiting substance described above can be used as it is, as the pharmaceutical product for treating cardiac injury. However, it is also possible to combine the p21-inhibiting substance with a pharmaceutically acceptable carrier to be used as the pharmaceutical composition. The pharmaceutically acceptable carrier which can be used may be a carrier used in a pharmaceutical product, such as a solvent, a buffer, a stabilizer, a vehicle or the like, and can be selected as appropriate, depending on the type of the p21-inhibiting substance and the dosage form of the pharmaceutical product.

In cases where the p21-inhibiting substance is a nucleic acid, the pharmaceutical product for treating cardiac injury may contain a reagent for delivering the nucleic acid to cells, such as a lipofection reagent, or a reagent for stabilizing the nucleic acid.

The subject to be administered with the pharmaceutical product according to the present invention is not particularly limited, as long as the effects of the present invention can be obtained. However, the subject is preferably a mammal, more preferably a primate such as a human or a rodent such as a mouse, and still more preferably a human.

The pharmaceutical product according to the present invention can be administered orally or parenterally, but is preferably administered locally to the site of cardiac injury. The dosage form of the pharmaceutical product is not particularly limited, and the pharmaceutical product can be administered, for example, in the form of an injectable liquid or an intravenous formulation.

The dose of the pharmaceutical product according to the present invention varies depending, for example, on the type of the p21-inhibiting substance, the age, sex and symptoms of the subject to be administered as well as the administration method, and can be selected as appropriate. However, the dose of the pharmaceutical product is, for example, from 0.01 mg to 1,000 mg per day, and preferably from 0.1 mg to 100 mg per day, as the amount of the p21-inhibiting substance as the active ingredient.

The pharmaceutical product may be administered in a single dose or multiple doses.

The pharmaceutical product according to the present invention may be used in combination with another therapeutic agent for cardiac injury.

### <2> Method According to Present Invention

One embodiment of the method according to the present invention is a method for promoting epicardial cell regeneration in vitro, the method including the step of inhibiting p21 in the epicardial cells.

Another embodiment of the method according to the present invention relates to a method for producing epicardial cells having an enhanced regenerative capacity, the method including:
A) the step of providing epicardial cells; and
B) the step of inhibiting p21 in the epicardial cells in the step A).

The phrase "promoting epicardial cell regeneration" as used herein is characterized, for example, by providing an improved viability of epicardial cells, an improved proliferative capacity of epicardial cells, an improved tissue repair capacity of the epicardium and/or an improved expression of a gene involved in the reactivation of the regenerative capacity of the epicardium. Further, the phrase "having an enhanced regenerative capacity" may mean, for example, an improved viability of epicardial cells, an improved proliferative capacity of epicardial cells, an improved tissue repair capacity of the epicardium and/or an improved expression of a gene involved in the reactivation of the regenerative capacity of the epicardium.

The epicardial cells may be epicardial cells collected from a mammal subject such as a human or a mouse, or may be epicardial cells obtained by inducing differentiation from pluripotent stem cells.

Examples of the pluripotent stem cells as used herein include, but not particularly limited to, embryonic stem (ES) cells, induced pluripotent stem (iPS) cells, embryonic stem (ntES) cells derived from a cloned embryo obtained by nuclear transfer, spermatogonial stem cells ("GS cells"), embryonic germ cell ("EG cells"), cultured fibroblasts and pluripotent cells (Muse cells) derived from bone marrow stem cells. Preferred pluripotent stem cells are iPS cells and ES cells. The pluripotent stem cells can be derived from any species, as long as the effects of the present invention can be obtained. However, the pluripotent stem cells are preferably derived from a mammal, more preferably derived from a primate such as a human or a rodent such as a mouse, and still more preferably derived from a human.

The methods for producing iPS cells are known in the art, and iPS cells can be produced, for example, by introducing reprogramming factors into any type of somatic cells. Examples of reprogramming factors as used herein include genes and gene products, such as Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3 and Glis1. These reprogramming factors may be used singly, or in combination. Examples of the combination of the reprogramming factors include combinations disclosed in the following literature: WO 2007/069666; WO 2008/118820; WO 2009/007852; WO 2009/032194; WO 2009/058413; WO 2009/057831; WO 2009/075119; WO 2009/079007; WO 2009/091659; WO 2009/101084; WO 2009/101407; WO 2009/102983; WO 2009/114949; WO 2009/117439; WO 2009/126250; WO 2009/126251; WO 2009/126655; WO 2009/157593; WO 2010/009015; WO 2010/033906; WO 2010/033920; WO 2010/042800; WO 2010/050626; WO 2010/056831; WO 2010/068955; WO 2010/098419; WO 2010/102267; WO 2010/111409; WO 2010/111422; WO 2010/115050; WO 2010/124290; WO 2010/147395; WO 2010/147612; Huangfu D, et al. (2008), Nat. Biotechnol., 26: 795-797; Shi Y, et al. (2008), Cell Stem Cell, 2: 525-528; Eminli S, et al. (2008), Stem Cells. 26: 2467-2474; Huangfu D, et al. (2008), Nat. Biotechnol. 26: 1269-1275; Shi Y, et al. (2008), Cell Stem Cell, 3, 568-574; Zhao Y, et al. (2008), Cell Stem Cell, 3: 475-479; Marson A, (2008), Cell Stem Cell, 3, 132-135; Feng B, et al. (2009), Nat. Cell Biol. 11: 197-203; R. L. Judson et al., (2009), Nat. Biotechnol., 27: 459-461; Lyssiotis C. A., et al. (2009), Proc Natl Acad Sci U S A. 106: 8912-8917; Kim J. B., et al. (2009), Nature. 461: 649-643; Ichida J. K., et al. (2009), Cell Stem Cell. 5: 491-503; Heng J. C., et al. (2010), Cell Stem Cell. 6: 167-74; Han J, et al. (2010), Nature. 463: 1096-100; Mali P, et al. (2010), Stem Cells. 28: 713-720; and Maekawa M, et al. (2011), Nature. 474: 225-9.

Examples of the somatic cells used for obtaining iPS cells include, but are not limited to: fetal somatic cells; neonatal somatic cells; and mature, healthy and diseased somatic cells; as well as primary cultured cells; passaged cells; and established cell lines. Specific examples of the somatic cells include: (1) tissue stem cells (somatic stem cells) such as neural stem cells, hematopoietic stem cells, mesenchymal stem cells and dental pulp stem cells; (2) tissue progenitor cells; and (3) differentiated cells such as blood cells (for example, peripheral blood cells and umbilical cord blood cells), lymphocytes, epithelial cells, endothelial cells, muscle cells, fibroblasts (for example, skin cells), hair cells, liver cells, gastric mucosal cells, intestinal cells, spleen cells, pancreatic cells (for example, pancreatic exocrine cells), brain cells, lung cells, renal cells and fat cells.

The epicardial cells derived from pluripotent stem cells may specifically be, for example, epicardial cells obtained by inducing differentiation from pluripotent stem cells in vitro. Any known method can be used as the method for obtaining epicardial cells by inducing differentiation from pluripotent stem cells. For example, the differentiation may be induced by adding an appropriate differentiation-inducing factor to the culture medium.

The method for obtaining epicardial cells by inducing differentiation from pluripotent stem cells is not particularly limited, and a known method can be used.

Specific examples of the method include a method in which an embryoid body (EB) is formed from pluripotent stem cells, and the EB is subjected to monolayer culture, in the presence of a GSK3 (Glycogen synthase kinase 3) inhibitor such as CHIR99021, SB431542 which is a TGF (Transforming growth factor)-β inhibitor, BMP4 (Bone morphogenetic protein 4) and VEGF (vascular endothelial cell growth factor).

In addition, it is also possible to use the methods disclosed in the following literature.
1. Witty A., et al. Nat Biotechnol. 2014 Oct; 32 (10): 1026-35. doi: 10.1038/nbt. 3002. PMID: 25240927.
2. Iyer D, et al. Development. 2015 Apr 15; 142 (8): 1528-41. doi: 10.1242/dev. 119271. PMID: 25813541.
3. Bao X, et al. Nat Biomed Eng. 2016; 1: 0003. doi: 10.1038/s41551-016-0003. PMID: 28462012.
4. Guadix JA, et al. Stem Cell Reports. 2017 Dec 12; 9 (6): 1754-1764. doi: 10.1016/j. stemcr. 2017. 10. 023. PMID: 29173898.

The epicardial cells obtained by inducing differentiation from pluripotent stem cells may be isolated and purified before use, or a cell population containing the epicardial cells can be used as it is.

The thus provided epicardial cells are subjected to a treatment for inhibiting p21.

The phrase "inhibiting p21" as used herein refers to inhibiting p21 using the "p21-inhibiting substance" described above. The step of inhibiting p21 in the epicardial cells may be carried out, for example, by adding the p21-inhibiting substance described above to the epicardial cells.

The epicardial cells can be treated with the p21-inhibiting substance, for example, by a method in which the p21-inhibiting substance is added to the culture liquid of the epicardial cells, and the cells are cultured for a period of time sufficient for epicardial cell regeneration, for example, from 5 hours to 10 days. The p21-inhibiting substance can be added at a concentration sufficient for epicardial cell regeneration, and the concentration of the p21-inhibiting substance can be set as appropriate depending on the type of the substance. The p21-inhibiting substance can be added additionally. Further, the p21-inhibiting substance can be removed from the culture medium, after the effect of inhibiting p21 has been achieved.

The epicardial cells having an enhanced regenerative capacity may be purified or concentrated before use.

The epicardial cells produced by the method according to the present invention may be used for the treatment of cardiac injury, for example, by transplanting the cells to a subject, as appropriate.

### EXAMPLES

The present invention will now be described more specifically, with reference to Examples. However, the present invention is in no way limited to the following embodiments.

### (Procedure)

### Induction of Differentiation into Human Epicardial Cells

A human iPS cell line (409B2) was used for the induction of differentiation into human epicardial cells. The human iPS cell line was maintained on radiation-treated murine fetal fibroblasts (MEF), in an ES cell culture medium (Primate ES Cell Medium (Cat. # RCHEMD001; manufactured by REPROCELL Inc.)) supplemented with 4 ng/mL of fibroblast growth factor (bFGF).

First, human iPS cells were prepared as a single cell suspension, and seeded on a low-adhesion, polyHEMA (poly-2-hydroxyethyl methacrylate)-coated dish, in order to form an embryoid body (EB). As an initial differentiation medium, StemPro-34 medium (Gibco) supplemented with 2 mM L-glutamine, 50 µM/mL ascorbic acid, 0.4 µM monothioglycerol and 150 mg/mL transferrin was used. To trigger differentiation, 10 µM Y27632 (ROCK inhibitor), 2 ng/mL human recombinant BMP4 and 0.5% Matrigel (manufactured by Corning Inc.) were added to the differentiation culture medium.

Twenty-four hours after the formation of the EB, 100% by volume of a cell culture medium supplemented with 4 ng/mL Activin A, 10 ng/mL human recombinant bFGF and 20 ng/mL human recombinant BMP4 was added. After 72 to 96 hours, the EB was dissociated into single cells, and the cells were reseeded (0.3 × 10⁵ cells/cm²) on a gelatin-coated dish, using a basic culture medium containing 3 mM CHIR99021, 10 µM SB431542, 30 ng/mL human recombinant BMP4 and 5 ng/mL human recombinant VEGF, to induce the differentiation into human epicardial cells. After 7 days and thereafter, the human epicardial cells obtained by inducing differentiation were maintained in a 10% fetal bovine serum (FBS)-containing DMEM (Dulbecco's modified Eagle's medium) supplemented with 10 µM SB431542. The resulting human epicardial cells obtained by inducing differentiation from the human iPS cell line are also referred to as "human iPS cell-derived fetal epicardium".

### Introduction of siRNA

In the siRNA transfection assay, the human iPS cell-derived fetal epicardium was seeded at a density of 0.8 × 10⁶ cells per 10 cm dish, and transfection was carried out using RNAiMax (Invitrogen). Twenty-four hours after the transfection, the culture medium was replaced with a fresh medium. A small interfering RNA (siRNA) (sense strand: CAAGGAGUCAGACAUUUUAtt (SEQ ID NO: 1), an anti-sense strand: UAAAAUGUCUGACUCCUUGtt (SEQ ID NO: 2)) targeting CDKN1A, and a negative control siRNA No.1 (4390843) derived from Ambion Silencer Select, were used in accordance with the manufacturer's instructions.

### Quantitative RT-PCR

RNAs were extracted using QIAzol lysis reagent (manufactured by QIAGEN N.V.), and cDNA was synthesized from the total RNA using ReverTra Ace qPCR RT Master Mix with gDNA Remover (manufactured by TOYOBO Co., Ltd.). Subsequently, quantitative RT-PCR was performed using Thunderbird SYBR qPCR Mix (manufactured by TOYOBO Co., Ltd.)), in One Step Real-Time PCR System (Applied Biosystems). The gene expression results were quantified by the ΔΔCt method, and normalized with GAPDH.

The primers used are as follows. CDKN1A: forward 5'-AGGGGACAGCAGAGGAAG-3' (SEQ ID NO: 3); reverse 5'-GCGTTTGGAGTGGTAGAAATCTG-3' (SEQ ID NO: 4), WT1: forward 5'-CAGCTTGAATGCATGACCTG-3' (SEQ ID NO: 5); reverse 5' - GATGCCGACCGTACAAGAGT-3' (SEQ ID NO: 6), and GAPDH: forward 5'-TGATGACATCAAGAAGGTGGTGAAG-3' (SEQ ID NO: 7); reverse 5'-TCCTTGGAGGCCATGTGGCCAT-3' (SEQ ID NO: 8).

### Western blotting

First, the cells were dissolved using M-PER Mammalian Protein Extraction Reagent (manufactured by Thermo Fisher Scientific Inc.) to obtain the total protein, and the total protein was quantified using Protein Assay BCA Kit (manufactured by Nacalai Tesque, Inc). For Western blotting, 8 µg of the total protein was loaded into each well of an SDS-PAGE gel. The antibodies used and the dilution ratios thereof are as follows: p21 (1:1000, Cat #2947; manufactured by Cell Signaling Technology, Inc.), WT1 (1:1000, ab89901; manufactured by Abcam Inc.), and β-actin (1:1000, A5441; manufactured by Sigma-Aldrich Co. LLC.).

### Cell Proliferation Curve Assay

The cell proliferation was evaluated by the time-course curve of the proliferation of the cells on a six-well plate. The cells were seeded at a density of 4 × 10⁴ cells per well, and the total number of cells was counted using trypan blue for 8 days at intervals of 24 hours. The cell proliferation curves were generated by plotting the number of cells against time. After seeding, the cells were fixed with 4% paraformaldehyde (PFA), and stained with crystal violet on the days the cells were counted. The values were represented as the cell proliferation rate of the cells proliferated in the presence of 10% FBS. "0%" refers to the number of cells on day 0.

### Colony Formation Assay

In the cell colony formation assay, 5,000 cells were seeded in a 10 cm-dish, and cultured for 10 days. Thereafter, the colony was fixed with 4% PFA, stained with crystal violet, and counted by Image-J.

### Wound-healing Assay

The cells were seeded on a six-well plate and cultured until 100% confluent. Subsequently, the cell monolayer was scratched with a pipette tip, to create a physical wound manually. The scratched area was imaged 0, 12, 16, 20 and 24 hours after scratching. The area without cells at the beginning, and the remaining area after 12, 16, 20 and 24 hours, were quantified by Image-J. Thereafter, the percentage of the scratched area into which migrating cells reassembled (the percentage of the wound-closed area) was calculated, with respect to the scratched area.

### RNA Sequencing

The data obtained by RNA sequencing were normalized using NOISeq. The data of the adult epicardium are published for the analysis, under the GSE accession number: GSE8484085. The primary data of the RNA sequencing were processed using RStudio, for mapping and gene expression analysis. The gene expression data for searching, loading and pre-processing were processed using the Bioconductor package, NOISeq, for performing the data analysis and the differential expression analysis of the RNA sequencing data. Heatmaps for clustering differentially expressed genes (DEG) were generated, using R script.

### CDKN1A Knockout (KO) Mouse

A CDKN1A knockout mouse was obtained from Jackson laboratory (#016565 strain). In the Cdknla gene of the knockout mouse strain, the second exon is replaced with a neomycin resistance cassette (Neo cassette). Further, a C57BL/6J mouse was used as an isogenic control with a similar genetic background.

### Epicardial Explant

The ex vivo explant assay was carried out using the heart of each 12-day-old murine embryo (E12). First, the heart of the murine E12 was placed on a gelatin-coated dish in a low-glucose DMEM supplemented with 15% FBS. A proliferated cultured product appeared within 24 hours, and the length of the explant was observed with a microscope after 7 days. The cells of the explant were maintained in a culture medium supplemented with 10 µM SB431542, in order to prevent spontaneous epithelial-mesenchymal transition (EMT).

### (Results) < Example 1 >

The human iPS cell-derived fetal epicardium was subjected to siRNA transfection assay, using a siRNA (siCDKN1A) targeting CDKN1A or a negative control siRNA (control), and the relative mRNA expression levels of CDKN1A were measured by quantitative RT-PCR. The results thereof are shown in FIG. 1.

Further, the amounts of the p21 protein and the WT1 protein were measured by Western blotting, for the human epicardial cells which had been subjected to the transfection assay using each siRNA. The results are shown in FIG. 2.

By the siCDKN1A, the expression level of CDKN1A at the mRNA level in the human iPS cell-derived fetal epicardium decreased to 50% or less as compared to that in the control, and a similar decrease was observed in the p21 protein level.

Subsequently, the cell proliferation curve assay was performed for the human iPS cell-derived fetal epicardium which had been subjected to the transfection assay using each siRNA, to generate a cell proliferation curve. The results are shown in FIG. 3. By decreasing the expression of CDKN1A, a significant increase in the proliferative capacity of the human epicardial cells was observed.

The colony formation assay was carried out in the same manner, in order to measure the viability of the human epicardial cells. The results are shown in FIG. 4. By decreasing the expression of CDKN1A, a significant increase in the viability of the human epicardial cells were observed.

Further, the wound-healing assay was performed for the human iPS cell-derived fetal epicardium which had been subjected to the transfection assay using each siRNA. The percentage of the wound-closed area over time is shown on the left in FIG. 5, the photographs of the wound area after 0 hours and 24 hours are shown on the right in in FIG. 5.

By decreasing the expression of CDKN1A in the human epicardial cells, a significant increase in wound healing capacity was observed during the period of time from 20 hours to 24 hours after scratching.

In addition, the transcriptome analysis for detecting the gene signature of cell quiescence was carried out by RNA sequencing (FIG. 6). The comparison of the human iPS cell-derived fetal epicardium and the adult epicardium is shown on the left in FIG. 6, and the comparison of the human iPS cell-derived fetal epicardium (CDKN1A expression inhibition: siCDKN1A group) which had been subjected to the transfection assay using the human iPS cell-derived fetal epicardium and the siCDKN1A, is shown on the right in FIG. 6.

In the group of genes in which the expression decreases in cell quiescence, the gene expression was lower in the order of the adult epicardium, the human iPS cell-derived fetal epicardium and the siCDKN1A group. Further, in the group of genes in which the expression increases in cell quiescence, the gene expression was higher in the order of the adult epicardium, the human iPS cell-derived fetal epicardium and siCDKN1A group. Accordingly, the cells are more highly quiescent in the order of the adult epicardium, the human iPS cell-derived fetal epicardium, and the siCDKN1A group. In other words, it has been revealed at the gene expression level that the cells are more released from quiescence in the siCDKN1A group, as compared to the human epicardium.

The above results revealed that it is possible to reactivate the regenerative capacity and to improve the wound healing capacity of human epicardial cells, by decreasing the expression of CDKN1A in the human epicardial cells.

### (Example 2)

The ex vivo explant assay of the CDKN1A knockout mouse was carried out. First, epicardial explants were prepared and cultured, ex vivo, using the hearts of E12 of the CDKN1A knockout mouse and the control mouse (C57BL/6J mouse). The details of the CDKN1A knockout mouse and the control mouse are as described in the section of "CDKN1A Knockout (KO) Mouse" described above. Further, the preparation and culture of each epicardial explant were carried out by the method described in the section of "Epicardial Explant" described above. Subsequently, the amounts of the p21 protein and the WT1 protein were measured by Western blotting, for each epicardial explant. The results are shown in FIG. 7.

In the epicardial explant obtained from the CDKN1A knockout mouse, the p21 protein was barely observed, and coincided with the effect of knocking out CDKN1A.

Further, the respective epicardial explants thus prepared were observed with a microscope 7 days after the culture, and the observed appearances are shown in FIG. 8. The epicardial explant obtained from the CDKN1A knockout mouse had a higher proliferation rate and showed an explant length almost twice, as compared to that obtained from the control, suggesting a high regenerative capacity.

The above results confirmed that it is possible to reactivate the regenerative capacity and to improve the wound healing capacity of epicardial cells, by decreasing or eliminating the expression of CDKN1A, also in the epicardial explant prepared using the heart of the murine E12.

## Claims

1. An agent for promoting epicardial cell regeneration comprising a p21-inhibiting substance.

2. The agent for promoting epicardial cell regeneration according to claim 1, wherein the p21-inhibiting substance is a substance that inhibits the expression of CDKN1A gene.

3. The agent for promoting epicardial cell regeneration according to claim 2, wherein the substance that inhibits the expression of the CDKN1A gene is a nucleic acid comprising a siRNA sequence that targets the CDKN1A gene.

4. The agent for promoting epicardial cell regeneration according to claim 3, wherein the siRNA sequence comprises a sense strand having the sequence of SEQ ID NO: 1, and an anti-sense strand having the sequence of SEQ ID NO: 2.

5. The agent for promoting epicardial cell regeneration according to claim 1 or 2, wherein the p21-inhibiting substance comprises a guide RNA for a CRISPR-Cas system, and the guide RNA for the CRISPR-Cas system is a guide RNA designed to target the CDKN1A gene.

6. A pharmaceutical composition for treating cardiac injury, the composition comprising the agent for promoting epicardial cell regeneration according to any one of claims 1 to 5.

7. A method for promoting epicardial cell regeneration in vitro, the method comprising the step of inhibiting p21 in the epicardial cells.

8. The method according to claim 7, wherein the step of inhibiting p21 is carried out by inhibiting the expression of CDKN1A gene.

9. The method according to claim 8, wherein the inhibition of the expression of the CDKN1A gene is achieved by introducing a siRNA into the epicardial cells.

10. The method according to claim 8, wherein the step of inhibiting p21 is carried out by introducing a guide RNA designed to target the CDKN1A gene and a CRISPR enzyme into the epicardial cells.

11. A method for producing epicardial cells having an enhanced regenerative capacity, the method comprising:
A) the step of providing epicardial cells; and
B) the step of inhibiting p21 in the epicardial cells provided in the step A).

12. The method according to claim 11, wherein the epicardial cells provided in the step A) are epicardial cells obtained by inducing differentiation from pluripotent stem cells.
